# EUROPEAN PATENT SPECIFICATION

(11) **EP 4 134 014 B1**
(45) Date of publication and mention of the grant of the patent: **07.05.2025**
(21) Application number: 20938315.7
(22) Date of filing: 21.10.2020
(51) Int. Cl.: A61B 17/064, A61B 17/068

(54) **FASTENING NAIL, FASTENING NAIL IMPLANTING DEVICE AND MEDICAL INSTRUMENT**
BEFESTIGUNGSNAGEL, BEFESTIGUNGSNAGEL-IMPLANTATIONSVORRICHTUNG UND MEDIZINISCHES INSTRUMENT
CLOU DE FIXATION, DISPOSITIF D'IMPLANTATION DE CLOU DE FIXATION ET INSTRUMENT MÉDICAL

(30) Priority: 29.05.2020 CN 202010482437
(43) Date of publication of application: 15.02.2023
(73) Proprietor: Creative Medtech (Suzhou) Co., Ltd, Suzhou, Jiangsu 215625 (CN)
(72) Inventor: DAI, Gaoxu, Beijing 102600 (CN); HAN, Wuen, Beijing 102600 (CN); YANG, Fan, Beijing 102600 (CN)
(74) Representative: Chung, Hoi Kan
(86) International application number: PCT/CN2020/122562
(87) International publication number: WO 2021/238036

(56) References cited:
- WO-A2-2014/064695
- CN-A- 107 847 320
- CN-A- 109 223 076
- CN-A- 110 740 692
- CN-A- 111 481 250
- CN-A- 111 481 251
- CN-A- 111 568 605
- CN-U- 210 447 086
- CN-U- 210 931 596
- CN-U- 212 326 481
- US-A1- 2012 022 557
- US-A1- 2012 022 557
- US-A1- 2017 143 340
- US-A1- 2018 049 875
- US-A1- 2019 175 344
- US-A1- 2019 282 229
- US-A1- 2020 179 114

## Description

### Technical Field

The present invention relates to the technical field of medical instrument, and in particular, to a fastening nail, a fastening nail implanting apparatus and a medical instrument.

### Background Art

During surgical treatment, it is usually required to use medical fastening nail, wherein the medical fastening nail is enabled to be nailed into human organ or skin tissue, so as to assist suturing. The fastening nail used in the prior art is usually in simple spiral structure, and the shifting fork is enabled to be inserted into a loop at one end of the fastening nail, then the shifting fork is rotated, thereby screwing the fastening nail into the human organ or skin tissue. However, in the process of using the structure of the prior art to perform the rotation on the fastening nail, the end ring at the rear end of the fastening nail, that is, the distal end often called during surgical treatment, is usually in a circular structure, and the shifting fork is usually in a square structure, it is difficult to firmly combine the two, so that the phenomenon of fastening nail being loosened are easy to occur during the rotation process, thereby delaying the completion of the operation, increasing the risk of the patient during the operation, and being unfavorable for postoperative wound healing of the patient. US 2012/022557 A1 discloses a multiple anchor delivery tool. US 2019/175344 A1 discloses a device system and a method for transcatheter treatment of valvular regurgitation. US 2019/282229 A1 and US 2017/143340 A1 each discloses a surgical fastener. US 2018/049875 A1 discloses an apparatus for repairing an atrioventricular valve.

### Summary

The invention is defined in the independent claims. Certain optional features of the invention are defined in the dependent claims.

One of the objectives of the present invention includes providing a fastening nail, which can solve the technical problem that the fastening nail is easy to be loosened from the shifting fork when the fastening nail is screwed into the human tissue by using the shifting fork in the prior art, thereby delaying the completion time of the operation, increasing the risk of the patient during the operation, and being unfavorable for postoperative wound healing of the patient.

Another object of the present invention includes providing a fastening nail implanting apparatus to solve the technical problem that the fastening nail is easy to be loosened from the shifting fork when the fastening nail is screwed into the human tissue by using the shifting fork in the prior art, thereby delaying the completion time of the operation, increasing the risk of the patient during the operation, and being unfavorable for postoperative wound healing of the patient.

Another object of the present invention includes providing a medical instrument, which can improve the tightness of the combination between the fastening nail and the fastening nail implanting apparatus, thereby avoiding the loosening of the fastening nail from the fastening nail implanting apparatus, and facilitating quickly implanting the fastening nail into the human body.

To achieve the above objectives, the embodiments of the present invention use the following technical solutions.

In a first aspect, embodiments of the present invention provide a fastening nail, which comprises a nail seat and a spiral part, wherein a front end of the spiral part is formed as a tip, a rear end of the spiral part is fixedly connected to a front end of the nail seat, and a first positioning part is provided on an end surface of a rear end of the nail seat, wherein an annular groove is provided on an outer peripheral surface of the nail seat, and is configured to be tied with a polymer suture for surgery.

In an optional embodiment, the first positioning part comprises a positioning hole or a positioning protrusion provided on the end surface of the rear end of the nail seat.

In an optional embodiment, the positioning hole is oval, triangular, rectangular, hexagonal or waist-shaped; or, a section of the positioning protrusion is oval, triangular, rectangular, hexagonal or waist-shaped.

In an optional embodiment, a threaded hole is provided on a bottom wall of the positioning hole, the threaded hole is configured to be matched with a conveying apparatus in a manner of screw thread, and the threaded hole extends in a direction of a rotation axial line for rotation of the fastening nail.

In an optional embodiment, a center of the first positioning part is located on the rotation axial line for rotation of the fastening nail; or a plurality of first positioning parts are provided, and the plurality of first positioning parts are distributed in an array manner on the end surface of the rear end of the nail seat around the rotation axial line for rotation of the fastening nail.

In a second aspect, embodiments of the present disclosure provide a fastening nail implanting apparatus, which is configured to implant the fastening nail into a human body; the fastening nail comprises a nail seat and a spiral part, wherein a front end of the spiral part is formed as a tip, a rear end of the spiral part is fixedly connected to a front end of the nail seat, and a first positioning part is provided on an end surface of a rear end of the nail seat;
the fastening nail implanting apparatus comprises a base and an implanting component, the implanting component comprises a conveying cable and an anchoring handle;
a rear end of the conveying cable is connected to the anchoring handle, and a front end of the conveying cable is provided with a second positioning part capable of being snapped with the first positioning part; and
the anchoring handle is installed on the base, and the anchoring handle is configured to be capable of driving the conveying cable to rotate under a first working condition, so as to drive the fastening nail snapped with the front end of the conveying cable to rotate synchronously with the conveying cable, and driving the conveying cable to release the fastening nail under a second working condition.

The conveying cable comprises a casing pipe, a rotating cable and a spring member, and the rotating cable passes through the casing pipe;
the anchoring handle comprises a housing, a first rotating knob and a second rotating knob, the housing is installed on the base, a front end of the first rotating knob is installed inside the housing, and the first rotating knob is capable of rotating relative to the housing;
a through hole penetrating through the first rotating knob in a front-rear direction is provided on the first rotating knob, a front end of the second rotating knob is arranged in a rear end of the first rotating knob, and the second rotating knob may rotate relative to the first rotating knob; and the rear end of the first rotating knob and the second rotating knob are both located outside the housing;
a rear end of the casing pipe is connected with the front end of the first rotating knob, and a rear end of the rotating cable is connected with the front end of the second rotating knob through the spring member; and
the second positioning part is provided at a front end of the casing pipe, a front end of the rotating cable is connected with a dowel pin, the threaded hole is provided on the end surface of the rear end of the nail seat, and an external thread matched with the threaded hole is provided on the outer peripheral surface of the dowel pin.

In an optional embodiment, a rear end of the dowel pin is connected to the front end of the rotating cable by a conical surface whose diameter gradually increases in a direction from the dowel pin to the rotating cable, and a conical abutting surface matched with the conical surface is provided on an inner wall surface of the front end of the casing pipe, the rotating cable is configured to drive the dowel pin to slide back and forth inside the casing pipe, and when the conical surface abuts against the conical abutting surface provided on the inner wall surface of the front end of the casing pipe, a front end of the dowel pin protrudes from the front end of the casing pipe and is matched with the threaded hole.

In an optional embodiment, an anti-reversion part is provided between the front end of the first rotating knob and the housing, and the anti-reversion part is configured to prevent the casing pipe from rotating in an opposite direction following a second rotating shell during the process that the dowel pin rotates in the opposite direction relative to the fastening nail and thus is disengaged from the fastening nail.

In an optional embodiment, the first rotating knob comprises an end connecting rod, a first shaft rod and a first rotating shell connected from front to back in sequence, and the end connecting rod and the first shaft rod are installed inside the housing; and the anti-reversion part comprises a pawl part and a ratchet structure provided on an outer peripheral surface of the first shaft rod, and a pawl of the pawl part and the ratchet structure are engaged with each other.

In an optional embodiment, the second rotating knob comprises a second shaft rod and a second rotating shell connected to a rear end of the second shaft rod, the second shaft rod is arranged inside the first rotating shell, and a rear end of the spring member is connected to a front end of the second shaft rod.

In an optional embodiment, an anti-rotation part is detachably connected between the first rotating knob and the second rotating knob, and the anti-rotation part is configured to make the first rotating knob and the second rotating knob rotate synchronously; and
a first positioning hole is provided on the end surface of the rear end of the first rotating knob, a second positioning hole penetrating through the second rotating knob in the front-rear direction is provided on the second rotating knob, and one end of the anti-rotation part extends into the first positioning hole after passing through the second positioning hole.

In an optional embodiment, the fastening nail implanting apparatus further comprises an advancing mechanism and a casing support, the advancing mechanism comprises an advancing handle, a first gear and a second gear;
the advancing handle is fixedly connected to the casing support, the rotating cable passes through a lumen of the casing support, and the front end of the first rotating knob is rotatably connected to a rear end of the lumen of the casing support; and
the first gear is provided on the advancing handle, the second gear is provided on the first rotating knob, and the first gear and the second gear are engaged with each other.

In an optional embodiment, the fastening nail implanting apparatus further comprises a first bending-adjustable sheathing canal mechanism, the first bending-adjustable sheathing canal mechanism comprises a first bending-adjustable handle and a first bending-adjustable sheathing canal, and the first bending-adjustable handle is installed on the base and located at front of the anchoring handle, the first bending-adjustable sheathing canal is installed on the first bending-adjustable handle, and the conveying cable passes through the first bending-adjustable sheathing canal and the first bending-adjustable handle.

In an optional embodiment, the first bending-adjustable handle comprises a bending-adjustable knob, a bending-adjustable screw and a bending-adjustable sliding block, wherein the bending-adjustable sliding block is sleeved on the bending-adjustable screw in a manner of screw thread, a bending-adjustable wire is connected between a front end of the first bending-adjustable sheathing canal and the bending-adjustable sliding block, and a rear end of the bending-adjustable screw is connected with the bending-adjustable knob, and the bending-adjustable knob is configured to drive the bending-adjustable screw to rotate, so that the bending-adjustable sliding block slides in a length direction of the bending-adjustable screw, thereby tightening or releasing the bending-adjustable wire, so as to adjust a degree of curvature of the first bending-adjustable sheathing canal.

In an optional embodiment, the fastening nail implanting apparatus further comprises a second bending-adjustable sheathing canal mechanism, the second bending-adjustable sheathing canal mechanism comprises a second bending-adjustable handle and a second bending-adjustable sheathing canal, wherein the second bending-adjustable handle is installed on the base and located between the first bending-adjustable handle and the anchoring handle, and the second bending-adjustable handle may slide back and forth relative to the base; and the second bending-adjustable sheathing canal is installed on the second bending-adjustable handle, and the second bending-adjustable sheathing canal passes through the first bending-adjustable handle and the first bending-adjustable sheathing canal, and the conveying cable passes through the second bending-adjustable sheathing canal and the second bending-adjustable handle.

In an optional embodiment, the first bending-adjustable sheathing canal mechanism further comprises a first support pipe fitting; and a first sealing gasket is provided inside the first bending-adjustable handle, and a via hole with an axial line extending in a front-rear direction is provided on the first sealing gasket; a front end of the first support pipe fitting is installed on a rear end of the first bending-adjustable handle, and the front end of the first support pipe fitting is inserted into the inside of the via hole, a lumen of the first support pipe fitting communicates with the first bending-adjustable sheathing canal, and the conveying cable passes through the first bending-adjustable sheathing canal and the lumen of the first support pipe fitting.

In an optional embodiment, the first bending-adjustable handle is rotatably installed on the base through a rotating structure; and/or the second bending-adjustable handle is rotatably installed on the base through the rotating structure, wherein the rotating structure comprises an upper shell, a lower shell and a fastener, the lower shell is fixed on the base, the upper shell and the lower shell are connected with each other by the fastener, and the first bending-adjustable handle and/or the second bending-adjustable handle is configured to be clamped between the upper shell and the lower shell or removed from a position between the upper shell and the lower shell in a state where the fastener is opened.

In a third aspect, embodiments of the present disclosure further provide a medical instrument, which comprises a fastening nail and a fastening nail implanting apparatus, wherein the fastening nail comprises a nail seat and a spiral part, a front end of the spiral part is formed as a tip, a rear end of the spiral part is fixedly connected to a front end of the nail seat, a first positioning part is provided on an end surface of a rear end of the nail seat, and an annular groove is provided on an outer peripheral surface of the nail seat, and is configured to be tied with a polymer suture for surgery. The fastening nail implanting apparatus is configured to implant the fastening nail into a human body; the fastening nail implanting apparatus comprises a base and an implanting component, and the implanting component comprises a conveying cable and an anchoring handle; a rear end of the conveying cable is connected to the anchoring handle, and a front end of the conveying cable is provided with a second positioning part capable of being snapped with the first positioning part; and the anchoring handle is installed on the base, and the anchoring handle is configured to be capable of driving the conveying cable to rotate under a first working condition, so as to drive the fastening nail snapped with the front end of the conveying cable to rotate synchronously with the conveying cable, and driving the conveying cable to release the fastening nail under a second working condition.

The beneficial effects of the fastening nail, the fastening nail implanting apparatus and the medical instrument provided by the embodiments of the present invention include the followings.

The first aspect of the embodiments of the present invention provides a fastening nail. Since the front end of the spiral part of the fastening nail is formed as a tip, the function of implanting the fastening nail into human organ or skin tissue can be realized by rotating the spiral part. In the above, in addition to the spiral part, the fastening nail also includes a nail seat, and a first positioning part is provided on the end surface of the rear end of the nail seat, so that the specific structure and shape of the first positioning part can be provided to be suitable for a shifting fork or other conveying apparatus for inputting the fastening nail into the human body, thereby improving the tightness of the combination between the fastening nail and the conveying apparatus, so as to avoid the loosening of the fastening nail from the conveying apparatus.

In addition, the fastening nail implanting apparatus provided by the second aspect of the embodiments of the present invention is configured to implant the fastening nail into the human body. Since the fastening nail implanting apparatus has the rear end of the conveying cable connected to the anchoring handle, the front end of the conveying cable is provided with a second positioning part capable of being snapped with the first positioning part; and the anchoring handle is installed on the base, and the anchoring handle is configured to be able to drive the conveying cable to rotate under the first working condition, thereby driving the fastening nail snapped with the front end of the conveying cable to rotate synchronously with the conveying cable, and drive the conveying cable to release the fastening nail under the second working condition, the fastening nail implanting apparatus is convenient to operate, and is conducive to quickly implanting the fastening nail into the human body, so as to shorten the operation time, and reduce the intraoperative risk, thereby facilitating the rapid postoperative healing of the patient.

The medical instrument provided by the third aspect of the embodiments of the present invention is not only convenient to operate, but also can improve the tightness of the combination between the fastening nail and the fastening nail implanting apparatus, which avoids the loosening of the fastening nail from the fastening nail implanting apparatus, facilitates the rapid implantation of fastening nail into the human body, so as to shorten the operation time, reduce the intraoperative risk, thereby facilitating the rapid postoperative healing of the patient.

### Brief Description of Drawings

In order to more clearly illustrate the specific embodiments of the present invention or the technical solutions in the prior art, the drawings needed to be used for the description of the specific embodiments or the prior art will be briefly introduced below, and apparently, the drawings in the following description show some embodiments of the present invention, and those ordinarily skilled in the art still could obtain other drawings in light of these drawings, without using any inventive efforts.
FIG. 1 is a schematic view of an overall structure of a fastening nail provided in an embodiment of the present invention;
FIG. 2 is a schematic view of an overall structure of a fastening nail implanting apparatus provided by an embodiment of the present invention in a state in which fastening nail is installed;
FIG. 3 is an enlarged view of a partial structure of A part in FIG. 2;
FIG. 4 is an explosion structural view of FIG. 3;
FIG. 5 is an explosion structural view in another view angle of A part in FIG. 2;
FIG. 6 is a schematic view of an overall structure of the fastening nail implanting apparatus provided by an embodiment of the present invention in a state in which an anchoring handle is not installed with a housing and the fastening nail is not installed;
FIG. 7 is an enlarged view of a partial structure of C part in FIG. 6;
FIG. 8 is an enlarged view of a partial structure of D part in FIG. 6;
FIG. 9 is an explosion view of a partial structure of the anchoring handle in FIG. 8;
FIG. 10 is a schematic view of an installation structure of a second bending-adjustable handle installed on a base in FIG. 2; and
FIG. 11 is a front sectional view of a partial structure of B part in FIG. 2.

Reference signs in drawings: 1-fastening nail; 11-nail seat; 110-first positioning part; 111-threaded hole; 120-second positioning part; 130-annular groove; 12-spiral part; 121-tip; 200-base; 310-conveying cable; 311-casing pipe; 312-rotating cable; 313-spring member; 314-dowel pin; 3141-conical surface; 320-anchoring handle; 321-first rotating knob; 3211-first rotating shell; 3212-first shaft rod; 3213-end connecting rod; 322-second rotating knob; 3221-second rotating shell; 3222-second shaft rod; 323-housing; 3231-sealing element; 400-anti-reversion part; 410-pawl part; 420-ratchet structure; 500-anti-rotation part; 600-advancing mechanism; 610-advancing handle; 620-first gear; 630-second gear; 700-casing support; 810-first bending-adjustable sheathing canal mechanism; 811-first bending-adjustable handle; 812-first bending-adjustable sheathing canal; 820-second bending-adjustable sheathing canal mechanism; 821-second bending-adjustable handle; 822-second bending-adjustable sheathing canal; 8100-bending-adjustable knob; 8200-bending-adjustable screw; 8300-bending-adjustable sliding block; 8111-first sealing gasket; 830-first support pipe fitting; 900-rotating structure; 910-upper shell; 920-lower shell; 930-fastener; 1000-front and rear adjustment structure; 1100-adjustment knob; 1200-gear; 1300-rack; 1400-position-limited plate.

### Detailed Description of Embodiments

In order to make the purpose, technical solutions and advantages of the embodiments of the present invention clearer, the technical solutions in the embodiments of the present invention will be described clearly and completely below in conjunction with the drawings in the embodiments of the present invention, obviously, the described embodiments are a part of the embodiments of the present invention, but not all of the embodiments. The components of the embodiments of the present invention generally described and shown in the drawings herein may be arranged and designed in various different configurations.

Therefore, the following detailed description of the embodiments of the present invention provided in the drawings is not intended to limit the claimed scope of the present invention, but merely represents selected embodiments of the present invention.

It should be noted that similar reference numerals and letters indicate similar items in the following drawings. Therefore, once a certain item is defined in one drawing, it does not need to be further defined and explained in the subsequent drawings.

In the description of the present invention, it should be noted that the orientation or positional relationship indicated by the terms "front", "rear", "inner", "outer" etc. are based on the orientation or positional relations as shown in the drawings, or it is the orientation or positional relationship that the product of the present invention is usually placed in use, merely for facilitating the description of the present invention and simplifying the description, rather than indicating or implying that related devices or elements have to be in the specific orientation, or configured or operated in a specific orientation, therefore, they should not be construed as limitations on the present invention. Besides, terms "first", and "second" etc. are only used to distinguish descriptions, but should not be construed as indicating or implying importance in relativity.

In the description of the present invention, it should also be noted that, unless otherwise definitely specified and limited, the terms "provide", "mount", and "connect" should be understood in a broad sense, for example, they can be fixed connection, detachable connection or integrated connection; they can be mechanical connection or electrical connection; they can be directly attached or indirectly attached by intermediate medium. Connection can be the internal communication between two components. For those ordinarily skilled in the art, the specific meaning of the above terms in the present invention can be understood according to the specific situation.

Some embodiments of the present invention will be described in detail below with reference to the drawings. The embodiments described below and characteristics in the embodiments may be combined with each other without conflict.

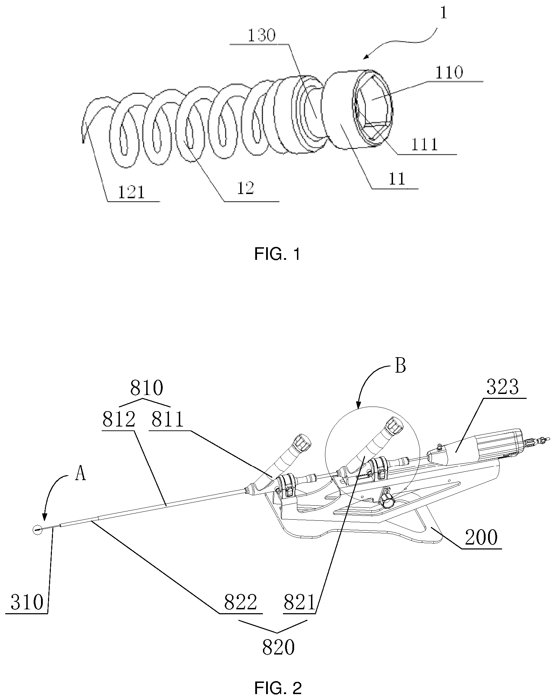

The embodiments of the present invention provide a fastening nail. Referring to FIG. 1, the fastening nail includes a nail seat 11 and a spiral part 12. The front end of the spiral part 12 is formed as a tip 121, the rear end of the spiral part 12 is fixedly connected to the front end of the nail seat 11, and a first positioning part 110 is provided on the end surface of the rear end of the nail seat 11.

In this embodiment, since the front end of the spiral part 12 is formed as a tip 121, the function of implanting the fastening nail 1 into a human organ or skin tissue can be realized by rotating the spiral part 12. In the above, in addition to the spiral part 12, the fastening nail 1 also includes a nail seat 11, and a first positioning part 110 is provided on the end surface of the rear end of the nail seat 11, so that the specific structure and shape of the first positioning part 110 can be provided to be suitable for a shifting fork or other conveying apparatus for inputting the fastening nail into the human body, thereby improving the tightness of the combination between the fastening nail 1 and the conveying apparatus, so as to avoid the loosening of the fastening nail 1 from the conveying apparatus.

Optionally, the spiral part 12 and the nail seat 11 are connected by means of riveting or welding. The spiral part 12 is formed by extending the material in a spiral shape, and the section shape of the material of the spiral part 12 is not specifically limited, for example, it may be a triangle, a circle or a rectangle.

The nail seat 11 is in a substantially columnar structure. Optionally, the end surface of the front end of the nail seat 11 and the rear end of the spiral part 12 are fixedly connected by means of riveting or welding.

The number of the first positioning part 110 is not specifically limited, and may be one or multiple, for example, one first positioning part 110 is provided, and the center of the first positioning part 110 is located on the rotation axial line for rotation of the fastening nail 1, for another example, a plurality of first positioning parts 110 may be provided, and the plurality of first positioning parts 110 are distributed in an array manner on the end surface of the rear end of the nail seat 11 around the rotation axial line for rotation of the fastening nail 1.

In an optional implementation of this embodiment, the above-mentioned first positioning part 110 may be formed in various specific structural forms, for example, but not limited to, the first positioning part 110 includes a positioning hole provided on the end surface of the rear end of the nail seat 11, and a positioning protrusion capable of being snapped with the positioning hole is provided at the front end of the conveying apparatus, so that the fastening nail 1 can be rotated through the positioning protrusion and the positioning hole, so as to implant the fastening nail 1 into human organ or skin tissue to assist suturing, wherein the positioning hole and the positioning protrusion are snapped with each other to ensure that the fastening nail 1 is not loosened from the conveying apparatus. Furthermore, the above-mentioned positioning holes may be one or more, and the corresponding positioning protrusions are in one-to-one correspondence to the positioning holes. In addition, optionally, the positioning hole may be of an ellipse or a polygon or the like, such as a triangle, a rectangle, a hexagon or a waist shape; in this embodiment, the positioning hole may be a waist-shaped hole.

Of course, the above-mentioned first positioning part 110 may also be formed in other specific structural forms, for example, but not limited to, the above-mentioned first positioning part 110 includes a positioning protrusion provided on the end surface of the rear end of the nail seat 11, and a positioning hole capable of being snapped with the positioning protrusion is provided at the front end of the conveying apparatus, so that the fastening nail 1 can be rotated through the positioning protrusion and the positioning hole, so as to implant the fastening nail 1 into human organ or skin tissue to assist the function of suturing and the like. Furthermore, the above-mentioned positioning protrusions may be one or more, and the corresponding positioning holes are in one-to-one correspondence to the above positioning protrusions. In addition, optionally, the section of above-mentioned positioning protrusion may be of an ellipse or a polygon or the like, such as a triangle, a rectangle, a hexagon or a waist shape. In addition, in order to facilitate the locking of the distance between the two fastening nails 1, as shown in FIG. 1, an annular groove 130 may be provided on the outer peripheral surface of the nail seat 11, by tying a polymer suture for surgery on the annular groove 130, and by knotting a suture on two fastening nails 1, such that the function of locking two fastening nails 1 is achieved, this method can be applied to an operation scenario where two parts of the tissue need to be closed, and the operation is convenient, which is beneficial to shorten the operation time.

In this embodiment, in order to further ensure the fixation and release of the fastening nail 1 and the conveying apparatus, a threaded hole 111 is provided on the end surface of the rear end of the nail seat 11, and the threaded hole 111 is configured to be matched with a conveying apparatus in a manner of screw thread. Optionally, the first positioning part 110 includes a positioning hole provided on the end surface of the rear end of the nail seat 11, the above-mentioned threaded hole 111 is provided on the bottom wall of the positioning hole, and the threaded hole 111 extends in a direction of the rotation axial line for rotation of the fastening nail 1.

The embodiments of the present invention further provide a fastening nail implanting apparatus, referring to FIG. 2 in conjunction with FIG. 1, the fastening nail implanting apparatus is configured to implant the fastening nail 1 provided by any one of the above-mentioned optional embodiments into a human body. The fastening nail implanting apparatus can be considered as a conveying apparatus in the above-mentioned embodiments.

Optionally, the fastening nail implanting apparatus may include a base 200 and an implanting component (not shown in the figure), and the implanting component includes a conveying cable 310 and an anchoring handle 320. Further, the rear end of the conveying cable 310 is connected to the anchoring handle 320, and the front end of the conveying cable 310 is provided with a second positioning part 120 capable of being snapped with the first positioning part 110. The anchoring handle 320 is installed on the base 200, and the anchoring handle 320 is configured to be capable of driving the conveying cable 310 to rotate under a first working condition, so as to drive the fastening nail 1 snapped with the front end of the conveying cable 310 to rotate synchronously with the conveying cable 310, and driving the conveying cable 310 to release the fastening nail 1 under a second working condition.

It should be noted that, in the embodiments of the present invention, if the first positioning part 110 includes a positioning hole provided on the end surface of the rear end of the nail seat 11, the second positioning part 120 includes a positioning protrusion provided at the front end of conveying cable 310, and the positioning protrusion and the positioning hole on the nail seat 11 are matched with each other. If the first positioning part 110 includes a positioning protrusion provided on the end surface of the rear end of the nail base 11, the second positioning part 120 includes a positioning hole provided at the front end of the conveying cable 310, and the positioning hole and the positioning protrusion on the nail seat 11 are matched with each other. In addition, the first positioning part 110 and the second positioning part 120 are in one-to-one correspondence, and the shapes are adapted to each other, and both may be oval, triangular, rectangular, hexagonal, or waist-shaped or the like.

Referring to FIG. 2 to FIG.7, in an optional implementation of this embodiment, optionally, the conveying cable 310 may include a casing pipe 311, a rotating cable 312 and a spring member 313, and the rotating cable 312 passes through the casing pipe 311.

Referring to FIG. 2, FIG. 8 and FIG. 9, the anchoring handle 320 may include a housing 323, a first rotating knob 321 and a second rotating knob 322. The housing 323 is installed on the base 200, the front end of the first rotating knob 321 is installed inside the housing 323, and the first rotating knob 321 may rotate relative to the housing 323. A through hole penetrating through the first rotating knob 321 in a front-rear direction is provided on the first rotating knob 321, a front end of the second rotating knob 322 is arranged in a rear end of the first rotating knob 321, it should be understood that the front end of the second rotating knob 322 extends into the rear end of the first rotating knob 321 through the above-mentioned through hole, and the second rotating knob 322 may rotate relative to the first rotating knob 321; and the rear end of the first rotating knob 321 and the second rotating knob 322 are both located outside the housing. The rear end of the casing pipe 311 is connected with the front end of the first rotating knob 321, and a rear end of the rotating cable 312 is connected with the front end of the second rotating knob 322 through the spring member 313. Continuing to refer to FIG. 2 to FIG. 7, the second positioning part 120 is provided at a front end of the casing pipe 311, a front end of the rotating cable 312 is connected with a dowel pin 314, the threaded hole 111 is provided on the end surface of the rear end of the nail seat 11. Optionally, the first positioning part 110 in this embodiment includes a positioning hole provided on the end surface of the rear end of the nail base 11, and the above-mentioned threaded hole 111 is provided on the bottom wall of the positioning hole. The second positioning part 120 includes a positioning protrusion protruding from the front end of the casing pipe 311, the positioning protrusion is matched with the positioning hole of the nail seat 11, the positioning protrusion is provided with an opening communicating with the inside of the casing pipe 311, wherein the opening is configured in such a way that the dowel pin 314 penetrates outward from the interior of the casing pipe 311. An external thread matched with the threaded hole 111 is provided on the outer peripheral surface of the dowel pin 314.

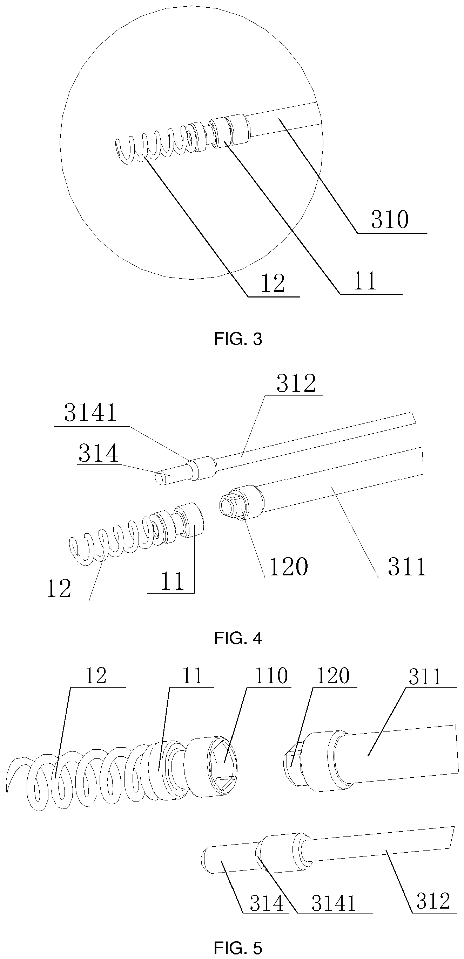

Further, as shown in FIG. 3, FIG. 4, FIG. 5 and FIG. 7, a rear end of the dowel pin 314 is connected to the front end of the rotating cable 312 by a conical surface 3141 whose diameter gradually increases in a direction from the dowel pin 314 to the rotating cable 312, and the other conical abutting surface (as not shown in the figure) matched with the conical surface 3141 is provided on an inner wall surface of the front end of the casing pipe 311. The rotating cable 312 may drive the dowel pin 314 to slide back and forth inside the casing pipe 311, and when the above-mentioned conical surface 3141 abuts against the other conical abutting surface provided on the inner wall surface of the front end of the casing pipe 311, the dowel pin 314 protrudes to the foremost end position relative to the casing pipe 311, at this time, a front end of the dowel pin 314 protrudes from the front end of the casing pipe 311 and is matched with the threaded hole 111 on the rear end of the nail seat 11.

When the anchoring handle 320 is used to implant the fastening nail 1 into the human body, the dowel pin 314 is threadedly connected to the inside of the threaded hole 111 provided on the end surface of the rear end of the nail seat 11 of the fastening nail 1, and the first positioning part 110 on the nail seat 11 of the fastening nail 1 is enabled to be snapped with the second positioning part 120 of the front end of the casing pipe 311, at this time, the conical abutting surface provided on the inner wall surface of the front end of the casing pipe 311 abuts against the conical surface 3141, the spring member 313 is in a stretched state, and the rear end surface of the nail seat 11 of the fastening nail 1 abuts against the front end surface of the casing pipe 311. By rotating the first rotating knob 321, the casing pipe 311 can be rotated, so that the casing pipe 311 drives the fastening nail 1 to rotate forward, so as to nail the front end of the fastening nail 1 into the human organ or skin tissue. After the rotation is in place, by rotating the second rotating knob 322 in an opposite direction, the dowel pin 314 can be enabled to rotate inside the casing pipe 311 relative to the screwed fastening nail 1, so that the dowel pin 314 can be separated from the fastening nail 1. At this time, the dowel pin 314 is retracted to the inside of the casing pipe 311 under the action of the elastic restoring force of the spring member 313. In this embodiment, by providing the spring member 313, the front end of the dowel pin 314 located outside the casing pipe 311 can be prevented from damaging the human body after the dowel pin 314 is separated from the fastening nail 1; and simultaneously, by providing the spring member 313, it can be ensured that before the dowel pin 314 is separated from the fastening nail 1, the dowel pin 314 always has a movement tendency of pulling the fastening nail 1 backward to make the rear end surface of the nail seat 11 of the fastening nail 1 abut against the front end surface of the casing pipe 311, so as to ensure that when the dowel pin 314 is separated from the fastening nail 1 , the fastening nail 1 is sleeved on the casing pipe 311, and the casing pipe 311 controls the fastening nail 1 not to rotate during the reverse rotation of the dowel pin 314 relative to the fastening nail 1, thereby ensuring that the dowel pin 314 can be smoothly disengaged from the fastening nail 1.

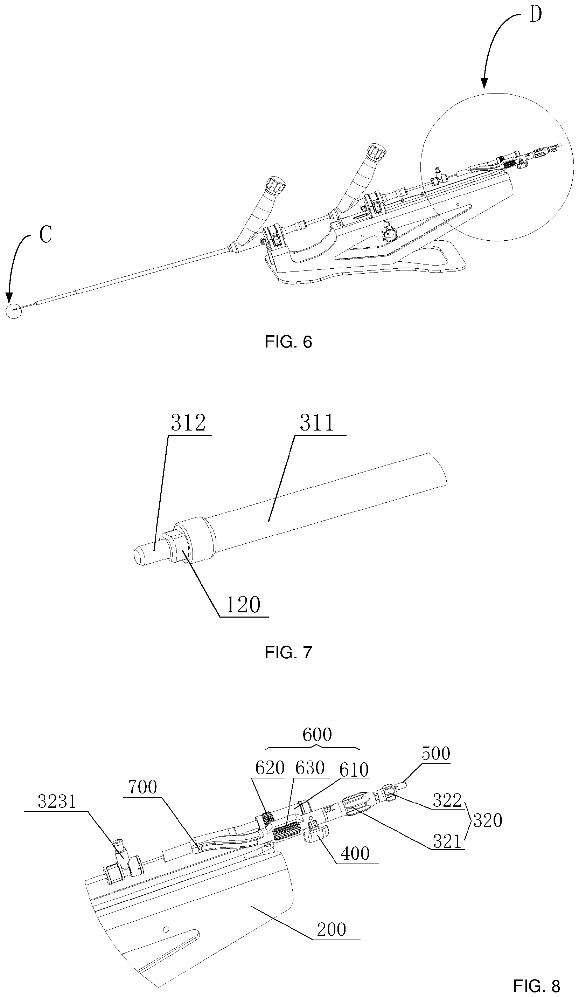

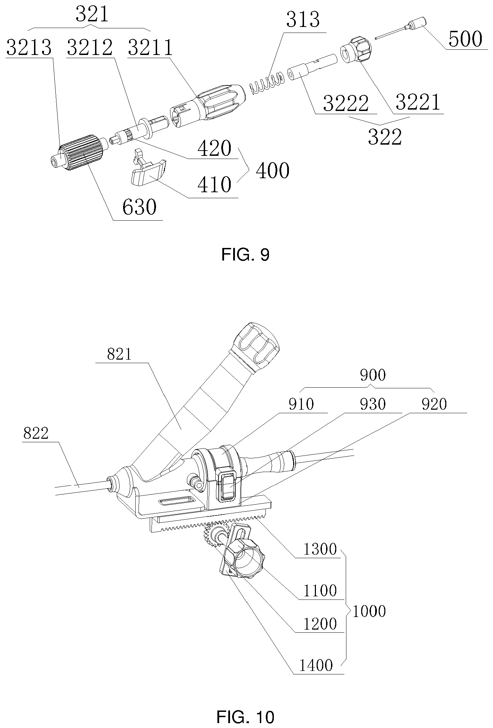

Referring to FIG. 8 and FIG. 9, in an optional embodiment of the present invention, an anti-reversion part 400 is provided between the front end of the first rotating knob 321 and the housing 323, and/or, an anti-rotation part 500 is detachably connected between the first rotating knob 321 and the second rotating knob 322. In the above, "and/or" means that only the former or only the latter or simultaneously both the former and the latter are provided in the structure of "an anti-reversion part 400 provided between the front end of the first rotating knob 321 and the housing 323" and the structure of "an anti-rotation part 500 detachably connected between the first rotating knob 321 and the second rotating knob 322".

There are various specific structural forms of the above anti-reversion part 400. Optionally, as shown in FIG. 8 and FIG. 9, the first rotating knob 321 may comprise an end connecting rod 3213, a first shaft rod 3212 and a first rotating shell 3211 connected from front to back in sequence, and the end connecting rod 3213 and the first shaft rod 3212 are mounted inside the housing 323. Optionally, the first rotating shell 3211 is detachably connected to one end of the first shaft rod 3212, and the other end of the first shaft rod 3212 is detachably connected to the end connecting rod 3213. In the above, one end of the first rotating shell 3211 is in a cylindrical structure, a snapping opening is provided on the peripheral wall of the cylindrical structure thereof, the peripheral wall of one end of the first shaft rod 3212 is provided with a snapping block, and one end of the first shaft rod 3212 extends into one end of the first rotating shell 3211, and the snapping block and the snapping opening are snapped with each other. The other end of the first shaft rod 3212 is protrudingly provided with a protruding part along axial line thereof, and the protruding part is matched with the snapping hole at one end of the end connecting rod 3213. Therefore, the end connecting rod 3213, the first shaft rod 3212 and the first rotating shell 3211 are firmly connected to each other, and are easy to disassemble and assemble with each other. In addition, the anti-reversion part 400 may comprise a pawl part 410 and a ratchet structure 420 provided on an outer peripheral surface of the first shaft rod 3212, and a pawl of the pawl part 410 and the ratchet structure 420 are engaged with each other. Referring to FIG. 2, one end of the pawl part 410 away from the ratchet structure 420 penetrates out of the housing 323. The second rotating knob 322 includes a second shaft rod 3222 and a second rotating shell 3221 connected to the rear end of the second shaft rod 3222. In the above, the second shaft rod 3222 is arranged inside the first rotating shell 3211, and the rear end of the spring member 313 is connected to the front end of the second shaft rod 3222. In an optional embodiment of the present invention, by providing the anti-reversion part 400, the casing pipe 311 can be enabled to only follow the second rotating shell 3221 to rotate in a forward direction, in the process of operating the second rotating shell 3221 to rotate, so that the dowel pin 314 rotates in an opposite direction relative to the fastening nail 1 and thus is disengaged from the fastening nail 1, it is ensured that the casing pipe 311 does not rotate in the opposite direction to follow the second rotating shell 3221, thereby ensuring that the dowel pin 314 is smoothly separated from the fastening nail 1. Of course, the structure of the above anti-reversion part 400 can also be other structures, for example, the above-mentioned pawl part 410 is replaced with a pawl provided on the inner wall of the housing 323, and the pawl is enabled to be engaged with the ratchet structure 420.

In addition, there are various specific structural forms of the above anti-rotation part 500, optionally, as shown in FIG. 8 and FIG. 9, an anti-rotation part 500 is detachably connected between the first rotating knob 321 and the second rotating knob 322. The anti-rotation part 500 is configured to make the first rotating knob 321 and the second rotating knob 322 rotate synchronously. Optionally, a first positioning hole can be provided on the end surface of the rear end of the first rotating knob 321, a second positioning hole penetrating through the second rotating knob 322 in the front-rear direction can be provided on the second rotating knob 322, and one end of the anti-rotation part 500 extends into the first positioning hole after passing through the second positioning hole, so as to avoid rotation of the second rotating knob 322 relative to the first rotating knob 321, when the first rotating knob 321 is rotated to implant the fastening nail 1 into the human organ or skin tissue. When the second rotating knob 322 needs to be rotated separately to make the dowel pin 314 separated from the fastening nail 1, the anti-rotation part 500 can be pulled out.

Continuing to refer to FIG. 8 and FIG. 9, in an optional embodiment of the present invention, the fastening nail implanting apparatus further may comprise an advancing mechanism 600 and a casing support 700, wherein the advancing mechanism 600 comprises an advancing handle 610, a first gear 620 and a second gear 630. The advancing handle 610 is fixedly connected to the casing support 700, the rotating cable 312 passes through a lumen of the casing support 700, and the front end of the first rotating knob 321 is rotatably connected to a rear end of the lumen of the casing support 700, an avoidance hole for avoiding the pawl part 410 is provided on the casing support 700. The first gear 620 is provided on the outer peripheral surface of the advancing handle 610, the second gear 630 is provided on the outer peripheral surface of the end connecting rod 3213 of the first rotating knob 321, and the first gear 620 and the second gear 630 are engaged with each other, and a window for exposing the second gear 630 is provided on the casing support 700. In an optional embodiment of the present invention, by providing the advancing mechanism 600, during the process of implanting the fastening nail 1 into the human organ or skin tissue through the first rotating knob 321, the casing support 700 can be enabled to slide forward relative to the house 323, so as to prevent the casing support 700 from providing a pulling force that pulls the fastening nail 1 backward, and to ensure that the fastening nail 1 is quickly screwed into human organ or skin tissue, optionally, the thread pitch of the external thread provided on the dowel pin 314 is equal to the tooth space of the first gear 620 provided on the advancing mechanism 600, so that the distance that the casing support 700 slides forward relative to the housing 323 is equal to the distance that the fastening nail 1 is nailed into the human organ or skin tissue.

In addition, as shown in FIG. 9, a sealing element 3231 is also provided inside the housing 323, and the casing pipe 311 passes through the sealing element 3231, so as to prevent blood leakage.

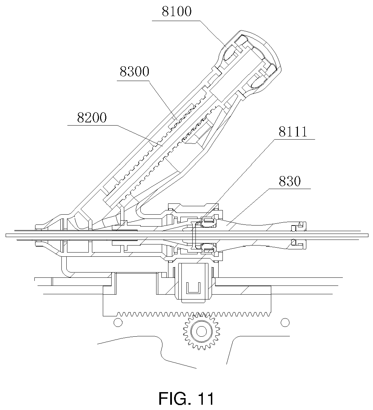

In addition, referring to FIG. 10 and FIG. 11, in conjunction with FIG. 2 and FIG. 6, and especially referring to FIG. 2, in an optional embodiment of the present invention, the fastening nail implanting apparatus further may comprise a first bending-adjustable sheathing canal mechanism 810, wherein the first bending-adjustable sheathing canal mechanism 810 may comprise a first bending-adjustable handle 811 and a first bending-adjustable sheathing canal 812, and the first bending-adjustable handle 811 is installed on the base 200 and located at front of the anchoring handle 320, the first bending-adjustable sheathing canal 812 is installed on the first bending-adjustable handle 811, and the conveying cable 310 passes through the first bending-adjustable sheathing canal 812 and the first bending-adjustable handle 811. Optionally, the first bending-adjustable handle 811 comprises a bending-adjustable knob 8100, a bending-adjustable screw 8200 and a bending-adjustable sliding block 8300, wherein the bending-adjustable sliding block 8300 is sleeved on the bending-adjustable screw 8200 in a manner of screw thread, a bending-adjustable wire is connected between a front end of the first bending-adjustable sheathing canal 812 and the bending-adjustable sliding block 8300, and a rear end of the bending-adjustable screw 8200 is connected with the bending-adjustable knob 8100, by rotating the bending-adjustable knob 8100, the bending-adjustable screw 8200 can be enabled to be rotated, so that the bending-adjustable sliding block 8300 slides in a length direction of the bending-adjustable screw 8200, thereby tightening or releasing the bending-adjustable wire, so as to achieve the function of adjusting a degree of curvature of the first bending-adjustable sheathing canal 812.

Further preferably, the fastening nail implanting apparatus further may comprise a second bending-adjustable sheathing canal mechanism 820, the second bending-adjustable sheathing canal mechanism 820 may comprise a second bending-adjustable handle 821 and a second bending-adjustable sheathing canal 822, wherein the second bending-adjustable handle 821 is installed on the base 200 and located between the first bending-adjustable handle 811 and the anchoring handle 320, and the second bending-adjustable handle 821 may slide back and forth relative to the base 200. The second bending-adjustable sheathing canal 822 is installed on the second bending-adjustable handle 821, and the second bending-adjustable sheathing canal 822 passes through the first bending-adjustable handle 811 and the first bending-adjustable sheathing canal 812, and the conveying cable 310 passes through the second bending-adjustable sheathing canal 822 and the second bending-adjustable handle 821. The bending-adjustable principle of the second bending-adjustable handle 821 is the same as that of the first bending-adjustable handle 811, thus, the second bending-adjustable handle 821 can be used to assist the first bending-adjustable handle 811 to perform secondary adjustment on the implanting angle, the first bending-adjustable handle 811 can make the implanting angle bended by 90 degrees before implantation, on this basis, and the second bending-adjustable handle 821 can be bended by 180 degrees before implantation. In addition, in this embodiment, there are various specific arrangement structure for the second bending-adjustable handle 821 capable of sliding back and forth relative to the base 200, for example, but not limited to, as shown in FIG. 10, the second bending-adjustable handle 821 is connected to the base 200 through a front and rear adjustment structure 1000, wherein the front and rear adjustment structure 1000 includes an adjustment knob 1100, a gear 1200, a rack 1300 and a position-limited plate 1400, the rack 1300 is provided at the bottom of the second bending-adjustable handle 821, one end of the adjustment knob 1100 is fixedly connected with a rotating shaft, and the rotating shaft passes through the position-limited long hole on the position-limited plate 1400, the gear 1200 is fixed on one end of the rotating shaft away from the adjustment knob 1100, the position-limited plate 1400 is fixed on the base 200, the rotating shaft can slide along the position-limited long hole and is located at two position-limited positions of the position-limited long hole, at one of the position-limited positions, the gear 1200 can be engaged with the rack 1300, and the rack 1300 extends in the front-rear direction, the adjustment knob 1100 is rotated, and then the second bending-adjustable handle 821 can move back and forth relative to the base 200, and at the other position-limited position, the gear 1200 can be disengaged from the rack 1300, and at this time, the second bending-adjustable handle 821 can be removed relative to the base 200; through the above structure, the first bending-adjustable handle 811 can be used to initially adjust the implanting position and the implanting angle, and then the second bending-adjustable handle 821 can be used to finely adjust the implanting position and the implanting angle, thereby increasing the precision of implanting position.

In addition, for the convenience of operation, in an optional embodiment of the present invention, the first bending-adjustable handle 811 may also be rotatably installed on the base 200 through the rotating structure 900; and/or the second bending-adjustable handle 821 may be rotatably installed on the base 200 through the rotating structure 900, wherein "and/or" means that only the former or only the latter or simultaneously both the former and the latter are provided in the structure of "the first bending-adjustable handle 811 rotatably installed on the base 200 through the rotating structure 900" and the structure of "the second bending-adjustable handle 821 rotatably installed on the base 200 through the rotating structure 900". Optionally, as shown in FIG. 10, the rotating structure 900 may include an upper shell 910, a lower shell 920 and a fastener 930, the lower shell 920 is fixed on the base 200, and the upper shell 910 and the lower shell 920 are connected with each other by the fastener 930, by opening the fastener 930, the bending-adjustable handle of the bending-adjustable sheathing canal mechanism can be enabled to be clamped between the upper shell 910 and the lower shell 920 or the bending-adjustable handle can be removed from a position between the upper shell 910 and the lower shell 920, and the bending-adjustable handle of the bending-adjustable sheathing canal mechanism can rotate between the upper shell 910 and the lower shell 920, so that the implanting angle can be adjusted more conveniently.

Furthermore, in order to facilitate the conveying cable 310 to smoothly pass through the bending-adjustable handle of the bending-adjustable sheathing canal, referring to FIG. 11, in an optional embodiment of the present invention, the first bending-adjustable sheathing canal mechanism 810 further comprise a first support pipe fitting 830; and a first sealing gasket 8111 is provided inside the first bending-adjustable handle 811, and a via hole with an axial line extending in a front-rear direction is provided on the first sealing gasket 8111; a front end of the first support pipe fitting 830 is installed on a rear end of the first bending-adjustable handle 811, and the front end of the first support pipe fitting 830 is inserted into the inside of the via hole, a lumen of the first support pipe fitting 830 communicates with the first bending-adjustable sheathing canal 812, and the conveying cable 310 passes through the first bending-adjustable sheathing canal 812 and the lumen of the first support pipe fitting 830, thus, the first support pipe fitting 830 can be used to open the via hole provided on the first sealing gasket 8111 in advance, so that the detachable process of the conveying cable 310 is simpler and more convenient, in addition, the second bending-adjustable sheathing canal mechanism 820 can further include a second support pipe fitting having the same structure as the first support pipe fitting 830.

The embodiments of the present invention also provide a medical instrument, the medical instrument comprises a fastening nail 1 and a fastening nail implanting apparatus provided in the above-mentioned embodiments, the fastening nail implanting apparatus is configured to implant the fastening nail 1 into a human body.

Finally, it should be noted that the above embodiments are only used to illustrate the technical solutions of the present invention, but not to limit them; although the present invention has been described in detail with reference to the foregoing embodiments, those ordinarily skilled in the art should understand that: the technical solutions described in the foregoing embodiments can still be modified, or some or all of the technical features thereof can be equivalently replaced.

### Industrial Applicability

To sum up, the fastening nail provided in the embodiments of the present invention can be applied to a shifting fork or other conveying apparatuses for inputting fastening nail into the human body, so as to improve the tightness of the combination between the fastening nail and the conveying apparatus, thereby avoiding the loosening of the fastening nail from the conveying apparatus. The fastening nail implanting apparatus provided by the embodiments of the present invention is convenient to operate, and is conducive to quickly implanting the fastening nail into the human body, so as to shorten the operation time, reduce the intraoperative risk, and facilitate the rapid postoperative healing of the patient. The medical instrument of the embodiment of the present invention is not only convenient to operate, but also can improve the tightness of the combination between the fastening nail and the fastening nail implanting apparatus, avoid the loosening of the fastening nail from the fastening nail implanting apparatus, and is conducive to the rapid implantation of fastening nail into the human body.

## Claims

1. A fastening nail, comprising a nail seat (11) and a spiral part (12),
wherein a front end of the spiral part (12) is formed as a tip (121), a rear end of the spiral part (12) is fixedly connected to a front end of the nail seat (11), and a first positioning part (110) is provided on an end surface of a rear end of the nail seat (11),
**characterized in that** an annular groove (130) is provided on an outer peripheral surface of the nail seat (11), and is configured to be tied with a polymer suture for surgery.

2. The fastening nail according to claim 1, wherein the first positioning part (110) comprises a positioning hole or a positioning protrusion provided on the end surface of the rear end of the nail seat (11).

3. The fastening nail according to claim 2, wherein the positioning hole is oval, triangular, rectangular, hexagonal or waist-shaped; or a section of the positioning protrusion is oval, triangular, rectangular, hexagonal or waist-shaped.

4. The fastening nail according to claim 2 or 3, wherein a threaded hole (111) is provided on a bottom wall of the positioning hole, the threaded hole (111) is configured to be matched with a conveying apparatus in a manner of screw thread, and the threaded hole (111) extends in a direction of a rotation axial line for rotation of the fastening nail (1).

5. The fastening nail according to any one of claims 1-4, wherein a center of the first positioning part (110) is located on the rotation axial line for rotation of the fastening nail (1); or
a plurality of first positioning parts (110) are provided, and the plurality of first positioning parts (110) are distributed in an array manner on the end surface of the rear end of the nail seat (11) around the rotation axial line for rotation of the fastening nail (1).

6. A fastening nail implanting apparatus, which is configured to implant the fastening nail (1) according to any one of claims 1 to 5 into a human body, wherein the fastening nail implanting apparatus comprises a base (200) and an implanting component, the implanting component comprises a conveying cable (310) and an anchoring handle (320);
a rear end of the conveying cable (310) is connected to the anchoring handle (320), and a front end of the conveying cable (310) is provided with a second positioning part (120) capable of being snapped with the first positioning part (110); and
the anchoring handle (320) is installed on the base (200), and the anchoring handle (320) is configured to be capable of driving the conveying cable (310) to rotate under a first working condition, so as to drive the fastening nail (1) snapped with the front end of the conveying cable (310) to rotate synchronously with the conveying cable (310), and driving the conveying cable (310) to release the fastening nail (1) under a second working condition,
wherein the conveying cable (310) comprises a casing pipe (311), a rotating cable (312) and a spring member (313), and the rotating cable (312) passes through the casing pipe (311);
the anchoring handle (320) comprises a housing (323), a first rotating knob (321) and a second rotating knob (322), the housing (323) is installed on the base (200), a front end of the first rotating knob (321) is installed inside the housing (323), and the first rotating knob (321) is capable of rotating relative to the housing (323);
a through hole penetrating through the first rotating knob (321) in a front-rear direction is provided on the first rotating knob (321), a front end of the second rotating knob (322) is arranged in a rear end of the first rotating knob (321), and the second rotating knob (322) can rotate relative to the first rotating knob (321); and the rear end of the first rotating knob (321) and the second rotating knob (322) are both located outside the housing (323);
a rear end of the casing pipe (311) is connected with the front end of the first rotating knob (321), and a rear end of the rotating cable (312) is connected with the front end of the second rotating knob (322) through the spring member (313); and
the second positioning part (120) is provided at a front end of the casing pipe (311), a front end of the rotating cable (312) is connected with a dowel pin (314), a threaded hole (111) is provided on the end surface of the rear end of the nail seat (11), and an external thread matched with the threaded hole (111) is provided on an outer peripheral surface of the dowel pin (314).

7. The fastening nail implanting apparatus according to claim 6, wherein an anti-reversion part (400) is provided between the front end of the first rotating knob (321) and the housing (323), and the anti-reversion part (400) is configured to prevent the casing pipe (311) from rotating in an opposite direction following a second rotating shell (3221) during a process that the dowel pin (314) rotates in an opposite direction relative to the fastening nail (1) and thus is disengaged from the fastening nail (1).

8. The fastening nail implanting apparatus according to any one of claims 6-7, wherein an anti-rotation part (500) is detachably connected between the first rotating knob (321) and the second rotating knob (322), and the anti-rotation part (500) is configured to make the first rotating knob (321) and the second rotating knob (322) rotate synchronously;
a first positioning hole is provided on an end surface of the rear end of the first rotating knob (321), a second positioning hole penetrating through the second rotating knob (322) in the front-rear direction is provided on the second rotating knob (322), and one end of the anti-rotation part (500) extends into the first positioning hole after passing through the second positioning hole.

9. The fastening nail implanting apparatus according to any one of claims 6-8, wherein the fastening nail implanting apparatus further comprises an advancing mechanism (600) and a casing support (700), the advancing mechanism (600) comprises an advancing handle (610), a first gear (620) and a second gear (630);
the advancing handle (610) is fixedly connected to the casing support (700), the rotating cable (312) passes through a lumen of the casing support (700), and the front end of the first rotating knob (321) is rotatably connected to a rear end of the lumen of the casing support (700); and
the first gear (620) is provided on the advancing handle (610), the second gear (630) is provided on the first rotating knob (321), and the first gear (620) and the second gear (630) are engaged with each other.

10. The fastening nail implanting apparatus according to any one of claims 6-9, wherein the fastening nail implanting apparatus further comprises a first bending-adjustable sheathing canal mechanism (810), the first bending-adjustable sheathing canal mechanism (810) comprises a first bending-adjustable handle (811) and a first bending-adjustable sheathing canal (812), and the first bending-adjustable handle (811) is installed on the base (200) and located at front of the anchoring handle (320), the first bending-adjustable sheathing canal (812) is installed on the first bending-adjustable handle (811), and the conveying cable (310) passes through the first bending-adjustable sheathing canal (812) and the first bending-adjustable handle (811).

11. The fastening nail implanting apparatus according to claim 10, wherein
the first bending-adjustable sheathing canal mechanism (810) further comprises a first support pipe fitting (830); and
a first sealing gasket (8111) is provided inside the first bending-adjustable handle (811), and a via hole with an axial line extending in a front-rear direction is provided on the first sealing gasket (8111); a front end of the first support pipe fitting (830) is installed on a rear end of the first bending-adjustable handle (811), and the front end of the first support pipe fitting (830) is inserted into inside of the via hole, a lumen of the first support pipe fitting (830) communicates with the first bending-adjustable sheathing canal (812), and the conveying cable (310) passes through the first bending-adjustable sheathing canal (812) and the lumen of the first support pipe fitting (830).

12. The fastening nail implanting apparatus according to claim 10 or 11, wherein at least one of the first bending-adjustable handle (811) and the second bending-adjustable handle (821) is rotatably installed on the base (200) through a rotating structure (900),
wherein the rotating structure (900) comprises an upper shell (910), a lower shell (920) and a fastener (930), the lower shell (920) is fixed on the base (200), the upper shell (910) and the lower shell (920) are connected by the fastener (930), and at least one of the first bending-adjustable handle (811) and the second bending-adjustable handle (812) is configured to be clamped between the upper shell (910) and the lower shell (920) or removed from a position between the upper shell (910) and the lower shell (920) in a state where the fastener (930) is opened.

13. A medical instrument, comprising a fastening nail (1) according to any one of claims 1 to 5, and a fastening nail implanting apparatus, wherein
the fastening nail implanting apparatus is configured to implant the fastening nail (1) into a human body; the fastening nail implanting apparatus comprises a base (200) and an implanting component, and the implanting component comprises a conveying cable (310) and an anchoring handle (320), wherein
a rear end of the conveying cable (310) is connected to the anchoring handle (320), and a front end of the conveying cable (310) is provided with a second positioning part (120) capable of being snapped with the first positioning part (110); and
the anchoring handle (320) is installed on the base (200), and the anchoring handle (320) is configured to be capable of driving the conveying cable (310) to rotate under a first working condition, so as to drive the fastening nail (1) snapped with a front end of the conveying cable (310) to rotate synchronously with the conveying cable (310), and driving the conveying cable (310) to release the fastening nail (1) under a second working condition.

## Patentansprüche

1. Ein Befestigungsnagel, der einen Nagelsitz (11) und einen spiralförmigen Teil (12) umfasst,
wobei ein vorderes Ende des spiralförmigen Teils (12) als eine Spitze (121) geformt ist, ein hinteres Ende des spiralförmigen Teils (12) fest mit einem vorderen Ende des Nagelsitzes (11) verbunden ist und ein erstes Positionierungsteil (110) an einer Endfläche eines hinteren Endes des Nagelsitzes (11) vorgesehen ist,
**dadurch gekennzeichnet, dass** eine ringförmige Rille (130) an einer äußeren Umfangsfläche des Nagelsitzes (11) vorgesehen ist und so konfiguriert ist, dass sie mit einem polymeren Nahtmaterial für die Chirurgie verbunden werden kann.

2. Der Befestigungsnagel gemäß Anspruch 1, wobei das erste Positionierungsteil (110) ein Positionierungsloch oder einen Positionierungsvorsprung umfasst, der an der Endfläche des hinteren Endes des Nagelsitzes (11) vorgesehen ist.

3. Der Befestigungsnagel gemäß Anspruch 2, wobei das Positionierungsloch oval, dreieckig, rechteckig, sechseckig oder taillenförmig ist; oder ein Abschnitt des Positionierungsvorsprungs oval, dreieckig, rechteckig, sechseckig oder taillenförmig ist.

4. Der Befestigungsnagel gemäß Anspruch 2 oder 3, wobei ein Gewindeloch (111) an einer Bodenwand des Positionierungslochs vorgesehen ist, das Gewindeloch (111) so konfiguriert ist, dass es mit einer Fördervorrichtung in der Art eines Schraubgewindes zusammenpasst, und das Gewindeloch (111) sich in Richtung einer axialen Rotationslinie zur Rotation des Befestigungsnagels (1) erstreckt.

5. Der Befestigungsnagel gemäß einem der Ansprüche 1-4, wobei ein Zentrum des ersten Positionierungsteils (110) auf der Rotationsachsenlinie für die Rotation des Befestigungsnagels (1) liegt; oder
mehrere erste Positionierungsteile (110) vorgesehen sind und die mehreren ersten Positionierungsteile (110) in Form eines Arrays auf der Endfläche des hinteren Endes des Nagelsitzes (11) um die axiale Rotationslinie für die Rotation des Befestigungsnagels (1) verteilt sind.

6. Ein Befestigungsnagel-Implantationsgerät, das konfiguriert ist, um den Befestigungsnagel (1) gemäß einem der Ansprüche 1 bis 5 in einen menschlichen Körper zu implantieren, wobei das Befestigungsnagel-Implantationsgerät eine Basis (200) und einen implantierenden Bestandteil umfasst, wobei der implantierende Bestandteil ein Förderkabel (310) und einen Verankerungsgriff (320) umfasst;
ein hinteres Ende des Förderkabels (310) mit dem Verankerungsgriff (320) verbunden ist, und ein vorderes Ende des Förderkabels (310) mit einem zweiten Positionierungsteil (120) versehen ist, das mit dem ersten Positionierungsteil (110) einschnappen kann; und
der Verankerungsgriff (320) an der Basis (200) installiert ist, und der Verankerungsgriff (320) so konfiguriert ist, dass er in der Lage ist, das Förderkabel (310) unter einer ersten Arbeitsbedingung zu drehen, um den Befestigungsnagel (1), der mit dem vorderen Ende des Förderkabels (310) eingerastet ist, anzutreiben, damit er sich synchron mit dem Förderkabel (310) dreht, und das Förderkabel (310) anzutreiben, um den Befestigungsnagel (1) unter einer zweiten Arbeitsbedingung freizugeben,
wobei das Förderkabel (310) ein Mantelrohr (311), ein Drehkabels (312) und ein Federelement (313) umfasst, und das Drehkabels (312) durch das Mantelrohr (311) verläuft;
der Verankerungsgriff (320) ein Gehäuse (323), einen ersten Drehknopf (321) und einen zweiten Drehknopf (322) umfasst, das Gehäuse (323) auf der Basis (200) installiert ist, ein vorderes Ende des ersten Drehknopfes (321) innerhalb des Gehäuses (323) installiert ist und der erste Drehknopf (321) in der Lage ist, sich relativ zum Gehäuse (323) zu drehen;
ein Durchgangsloch, das den ersten Drehknopf (321) in einer Richtung von vorne nach hinten durchdringt, an dem ersten Drehknopf (321) vorgesehen ist, ein vorderes Ende des zweiten Drehknopfes (322) in einem hinteren Ende des ersten Drehknopfes (321) angeordnet ist und der zweite Drehknopf (322) relativ zu dem ersten Drehknopf (321) drehbar ist; und das hintere Ende des ersten Drehknopfes (321) und der zweite Drehknopf (322) beide außerhalb des Gehäuses (323) angeordnet sind;
ein hinteres Ende des Mantelrohrs (311) mit dem vorderen Ende des ersten Drehknopfes (321) verbunden ist, und ein hinteres Ende des Drehkabels (312) mit dem vorderen Ende des zweiten Drehknopfes (322) durch das Federelement (313) verbunden ist; und
das zweite Positionierungsteil (120) an einem vorderen Ende des Mantelrohrs (311) vorgesehen ist, ein vorderes Ende des Drehkabels (312) mit einem Passstift (314) verbunden ist, ein Gewindeloch (111) an der Endfläche des hinteren Endes des Nagelsitzes (11) vorgesehen ist und ein mit dem Gewindeloch (111) zusammenpassendes Außengewinde an einer äußeren Umfangsfläche des Passstifts (314) vorgesehen ist.

7. Das Befestigungsnagel-Implantationsgerät gemäß Anspruch 6, wobei ein Anti-Reversions-Teil (400) zwischen dem vorderen Ende des ersten Drehknopfes (321) und dem Gehäuse (323) vorgesehen ist, und das Anti-Reversions-Teil (400) so konfiguriert ist, dass es verhindert, dass sich das Mantelrohr (311) in eine entgegengesetzte Richtung dreht, die einer zweiten Drehhülse (3221) folgt, während eines Vorgangs, bei dem sich der Passstift (314) in eine entgegengesetzte Richtung relativ zu dem Befestigungsnagel (1) dreht und somit von dem Befestigungsnagel (1) gelöst wird.

8. Das Befestigungsnagel-Implantationsgerät gemäß einem der Ansprüche 6-7, wobei ein Ende des Antirotationsteils (500) lösbar zwischen dem ersten Drehknopf (321) und dem zweiten Drehknopf (322) verbunden ist, und das Ende des Antirotationsteils (500) so konfiguriert ist, dass es den ersten Drehknopf (321) und den zweiten Drehknopf (322) dazu bringt, sich synchron zu drehen;
ein erstes Positionierungsloch an einer Endfläche des hinteren Endes des ersten Drehknopfes (321) vorgesehen ist, ein zweites Positionierungsloch, das den zweiten Drehknopf (322) in der Richtung von vorne nach hinten durchdringt, an dem zweiten Drehknopf (322) vorgesehen ist, und ein Ende des Antirotationsteils (500) sich in das erste Positionierungsloch erstreckt, nachdem es durch das zweite Positionierungsloch hindurchgegangen ist.

9. Das Befestigungsnagel-Implantationsgerät gemäß einem der Ansprüche 6-8, wobei das Befestigungsnagel-Implantationsgerät ferner einen Vorschubmechanismus (600) und einen Gehäuseträger (700) umfasst, wobei der Vorschubmechanismus (600) einen Vorschubgriff (610), ein erstes Zahnrad (620) und ein zweites Zahnrad (630) umfasst;
der Vorschubgriff (610) fest mit dem Gehäuseträger (700) verbunden ist, das Drehkabel (312) durch ein Lumen des Gehäuseträgers (700) verläuft und das vordere Ende des ersten Drehknopfes (321) drehbar mit einem hinteren Ende des Lumens des Gehäuseträgers (700) verbunden ist; und
das erste Zahnrad (620) an dem Vorschubgriff (610) vorgesehen ist, das zweite Zahnrad (630) an dem ersten Drehknopf (321) vorgesehen ist und das erste Zahnrad (620) und das zweite Zahnrad (630) miteinander in Eingriff sind.

10. Das Befestigungsnagel-Implantationsgerät gemäß einem der Ansprüche 6-9, wobei das Befestigungsnagel-Implantationsgerät ferner einen erste biegeverstellbare Umhüllungskanalmechanismus (810) umfasst, wobei der biegeverstellbare Umhüllungskanalmechanismus (810) einen ersten biegeverstellbaren Griff (811) und einen ersten biegeverstellbare Umhüllungskanal (812) umfasst, und der erste biegeverstellbare Griff (811) auf der Basis (200) installiert ist und sich vor dem Verankerungsgriff (320) befindet, der erste biegeverstellbare Umhüllungskanal (812) auf dem ersten biegeverstellbaren Griff (811) installiert ist, und das Förderkabel (310) durch den ersten biegeverstellbaren Umhüllungskanal (812) und den ersten biegeverstellbaren Griff (811) verläuft.

11. Das Befestigungsnagel-Implantationsgerät gemäß Anspruch 10, wobei
der ersten biegeverstellbaren Umhüllungskanalmechanismus (810) ferner eine erste Stützrohrbefestigung (830) umfasst; und
eine erste versiegelte Dichtung (8111) innerhalb des ersten biegeverstellbaren Griffs (811) vorgesehen ist und ein Durchgangsloch mit einer axialen Linie, die sich in einer Vorwärts-Rückwärts-Richtung erstreckt, an der ersten versiegelten Dichtung (8111) vorgesehen ist; ein vorderes Ende der ersten Stützrohrbefestigung (830) an einem hinteren Ende des ersten biegeverstellbaren Griffs (811) installiert ist und das vordere Ende der ersten Stützrohrbefestigung (830) in das Innere des Durchgangslochs eingesteckt ist, ein Lumen der ersten Stützrohrbefestigung (830) mit dem ersten biegeverstellbaren Umhüllungskanal (812) in Verbindung steht und das Förderkabel (310) durch den ersten biegeverstellbaren Umhüllungskanal (812) und das Lumen der ersten Stützrohrbefestigung (830) verläuft.

12. Das Befestigungsnagel-Implantationsgerät gemäß Anspruch 10 oder 11, wobei mindestens einer der ersten biegeverstellbaren Handgriffe (811) und der zweite biegeverstellbare Handgriff (821) über eine Drehstruktur (900) drehbar an der Basis (200) angebracht ist,
wobei die Drehstruktur (900) eine obere Schale (910), eine untere Schale (920) und ein Befestigungselement (930) umfasst, die untere Schale (920) an der Basis (200) befestigt ist, die obere Schale (910) und die untere Schale (920) durch das Befestigungselement (930) verbunden sind, und mindestens einer von dem ersten biegeverstellbaren Griff (811) und dem zweiten biegeverstellbaren Griff (812) so konfiguriert ist, dass er in einem Zustand, in dem der Befestigungselement (930) geöffnet ist, zwischen der oberen Schale (910) und der unteren Schale (920) eingeklemmt oder aus einer Position zwischen der oberen Schale (910) und der unteren Schale (920) entnommen werden kann.

13. Medizinisches Gerät, umfassend einen Befestigungsnagel (1) gemäß einem der Ansprüche 1 bis 5 und ein Befestigungsnagel-Implantationsgerät, wobei
das Befestigungsnagel-Implantationsgerät so konfiguriert ist, dass es den Befestigungsnagel (1) in einen menschlichen Körper implantiert; das Befestigungsnagel-Implantationsgerät eine Basis (200) und einen implantierenden Bestandteil umfasst, und der implantierende Bestandteil ein Förderkabel (310) und einen Verankerungsgriff (320) umfasst, wobei
ein hinteres Ende des Förderkabels (310) mit dem Verankerungsgriff (320) verbunden ist, und ein vorderes Ende des Förderkabels (310) mit einem zweiten Positionierungsteil (120) versehen ist, das mit dem ersten Positionierungsteil (110) einschnappen kann; und
der Verankerungsgriff (320) an der Basis (200) installiert ist und der Verankerungsgriff (320) so konfiguriert ist, dass er in der Lage ist, das Förderkabel (310) anzutreiben, um sich unter einer ersten Arbeitsbedingung zu drehen, um so den Befestigungsnagel (1) anzutreiben, der mit einem vorderen Ende des Förderkabels (310) eingerastet ist, um sich synchron mit dem Förderkabel (310) zu drehen, und das Förderkabel (310) anzutreiben, um den Befestigungsnagel (1) unter einer zweiten Arbeitsbedingung freizugeben.

## Revendications

1. Un clou de fixation, comprenant un siège du clou (11) et une partie hélicoïdale (12), dans lequel l'extrémité avant de la partie hélicoïdale (12) est formée en une pointe (121), l'extrémité arrière de la partie hélicoïdale (12) est fixée de manière permanente à l'extrémité avant du siège du clou (11), et une première partie de positionnement (110) est prévue sur la surface d'extrémité de l'arrière du siège du clou (11), **caractérisé en ce que** le siège du clou (11) est doté d'un rainurage annulaire (130) sur sa surface périphérique externe, conçu pour être lié à une suture en polymère chirurgicale.

2. Clou de fixation selon la revendication 1, dans lequel la première partie de positionnement (110) comprend un orifice de positionnement ou une protubérance de positionnement prévue sur la surface d'extrémité de l'arrière du siège du clou (11).

3. Clou de fixation selon la revendication 2, dans lequel l'orifice de positionnement est de forme ovale, triangulaire, rectangulaire, hexagonale ou en forme de taille ; ou bien une section de la protubérance de positionnement est de forme ovale, triangulaire, rectangulaire, hexagonale ou en forme de taille.

4. Clou de fixation selon la revendication 2 ou 3, dans lequel un trou fileté (111) est prévu sur la paroi inférieure de l'orifice de positionnement, ce trou fileté étant conçu pour s'accorder avec un dispositif de convoyage par filetage, et s'étendant dans la direction de l'axe de rotation du clou (1).

5. Clou de fixation selon l'une quelconque des revendications 1 à 4, dans lequel le centre de la première partie de positionnement (110) est situé sur l'axe de rotation du clou de fixation (1) ; ou bien plusieurs parties de positionnement (110) sont prévues et disposées de manière matricielle sur la surface d'extrémité de l'arrière du siège du clou (11) autour de l'axe de rotation du clou de fixation (1).

6. Dispositif d'implantation de clou, conçu pour implanter le clou de fixation (1) selon l'une quelconque des revendications 1 à 5 dans un corps humain, dans lequel le dispositif comprend une base (200) et un composant d'implantation, ce composant comprenant un câble de convoyage (310) et une poignée d'ancrage (320);
L'extrémité arrière du câble de convoyage (310) est reliée à la poignée d'ancrage (320) et l'extrémité avant du câble de convoyage (310) est munie d'une deuxième partie de positionnement (120) susceptible de s'emboîter avec la première partie de positionnement (110) ; par ailleurs
la poignée d'ancrage (320) est installée sur la base (200) et est conçue pour entraîner le câble de convoyage (310) à tourner dans une première condition de fonctionnement, de manière à faire tourner synchroniquement le clou de fixation (1) emboîté à l'extrémité avant du câble de convoyage (310), puis à entraîner le câble pour libérer le clou de fixation (1) dans une seconde condition de fonctionnement,
dans lequel le câble de convoyage (310) comprend un tube de gaine (311), un câble rotatif (312) et un élément ressort (313), le câble rotatif (312) passant à travers le tube (311) ;
la poignée d'ancrage (320) comprend un boîtier (323), un premier bouton rotatif (321) et un second bouton rotatif (322) ; le boîtier (323) est installé sur la base (200), l'extrémité avant du premier bouton rotatif (321) est placée à l'intérieur du boîtier (323) et le premier bouton rotatif (321) peut tourner par rapport au boîtier (323) ;
un orifice traversant, perçant le premier bouton rotatif (321) dans la direction avant-arrière, est prévu sur le premier bouton rotatif (321) ; une extrémité avant du second bouton (322) est disposée à l'extrémité arrière du premier bouton rotatif (321) et le second bouton (322) peut tourner par rapport au premier ; par ailleurs, l'extrémité arrière du premier bouton rotatif (321) et le second bouton (322) se situent tous deux à l'extérieur du boîtier (323) ;
l'extrémité arrière du tube de gaine (311) est reliée à l'extrémité avant du premier bouton rotatif (321), et l'extrémité arrière du câble rotatif (312) est connectée à l'extrémité avant du second bouton (322) via l'élément ressort (313) ;
la deuxième partie de positionnement (120) est prévue à l'extrémité avant du tube de gaine (311), une extrémité avant du câble rotatif (312) est reliée à une goupille (314), un trou fileté (111) est prévu sur la surface d'extrémité de l'arrière du siège du clou (11) et un filetage externe correspondant au trou fileté(111) est disposé sur la surface périphérique externe de la goupille (314).

7. Le dispositif d'implantation de clou selon la revendication 6, dans lequel une partie anti-retour (400) est prévue entre l'extrémité avant du premier bouton rotatif (321) et le boîtier (323), cette partie étant conçue pour empêcher le tube (311) de tourner dans le sens inverse lors d'une rotation du clou de fixation (1) par laquelle la goupille (314) tourne dans le sens opposé et se désengage du clou de fixation (1), suite à l'action d'une seconde coque rotative (3221).

8. Le dispositif d'implantation de clou selon l'une quelconque des revendications 6-7, dans lequel une partie anti-rotation (500) est connectée de manière détachable entre le premier bouton rotatif (321) et le second (322), et est conçue pour faire tourner ces deux boutons de manière synchronisée ;
un premier orifice de positionnement est prévu sur la surface d'extrémité arrière du premier bouton rotatif (321), un second orifice traversant, dans la direction avant-arrière, est prévu sur le second bouton (322), et une extrémité de la partie anti-rotation (500) s'insère dans le premier orifice après être passée par le second.

9. Le dispositif d'implantation de clou selon l'une quelconque des revendications 6-8, dans lequel le dispositif comprend en outre un mécanisme d'avancement (600) et un support de gaine (700), le mécanisme (600) comprenant une poignée d'avancement (610), un premier engrenage (620) et un second engrenage (630) ;
la poignée d'avancement (610) est fixée au support (700), le câble rotatif (312) passe par une lumière du support (700) et l'extrémité avant du premier bouton rotatif (321) est reliée de manière rotative à l'extrémité arrière de cette lumière ; de plus,
le premier engrenage (620) est disposé sur la poignée d'avancement (610), le second engrenage (630) sur le premier bouton rotatif (321), et les deux engrenages s'engagent l'un avec l'autre.

10. L'appareil d'implantation de clous de fixation selon l'une des revendications 6 à 9, dans lequel l'appareil d'implantation de clous de fixation comprend en outre un premier mécanisme de canal de gainage ajustable (810), le premier mécanisme de canal de gainage ajustable (810) comprend une première poignée ajustable (811) et un premier canal de gainage réglable en flexion (812), et la première poignée ajustable (811) est installée sur la base (200) et située à l'avant de la poignée d'ancrage (320), le premier canal de gainage réglable en flexion (812) est installé sur la première poignée ajustable (811), et le câble de convoyage (310) passe à travers le premier canal de gainage réglable en flexion (812) et la première poignée ajustable (811).

11. Le dispositif d'implantation de clou selon la revendication 10, dans lequel le premier mécanisme de canal de gainage ajustable (810) comprend en outre une première fixation de tuyau de support (830) ;
un premier joint d'étanchéité (8111) est prévu à l'intérieur de la première poignée ajustable (811) et un orifice traversant, dont l'axe s'étend dans la direction avant-arrière, est prévu sur ce joint un premier joint d'étanchéité(8111) ; une extrémité avant de la fixation de tuyau de support (830) est installée sur l'extrémité arrière de la poignée ajustable (811) et cette extrémité est insérée dans l'orifice, la lumière de la fixation de tuyau de support (830) communiquant avec le canal de gainage ajustable (812), et le câble de convoyage (310) passe à la fois par le canal (812) et par la lumière de la fixation de tuyau de support (830).

12. Le dispositif d'implantation de clou selon la revendication 10 ou 11, dans lequel au moins l'une des poignées ajustables en flexion (811) ou seconde poignée ajustable (821) est montée rotativement sur la base (200) via une structure rotative (900),
laquelle comprend une coque supérieure (910), une coque inférieure (920) et un dispositif de fixation (930) ; la coque inférieure (920) étant fixée sur la base (200) et la coque supérieure (910) reliée à la coque inférieure (920) par le dispositif de fixation (930), et au moins l'une des poignées (811) ou seconde poignée ajustable (812) est conçue pour être serrée entre la coque supérieure (910) et la coque inférieure (920) ou pour être retirée de l'espace entre ces deux coques lorsque le dispositif de fixation (930) est ouvert.

13. Un instrument médical, comprenant un clou de fixation (1) selon l'une quelconque des revendications 1 à 5, et un dispositif d'implantation de clou, dans lequel
le dispositif est conçu pour implanter le clou de fixation (1) dans le corps humain ; ce dispositif comprend une base (200) et un composant d'implantation, lequel comprend un câble de convoyage (310) et une poignée d'ancrage (320),
L'extrémité arrière du câble de convoyage (310) est reliée à la poignée d'ancrage (320) et l'extrémité avant du câble de convoyage (310) est munie d'une deuxième partie de positionnement (120) susceptible de s'emboîter avec la première partie de positionnement (110) ; par ailleurs
la poignée d'ancrage (320) étant installée sur la base (200) et étant conçue pour entraîner le câble de convoyage (310) à tourner dans une première condition de fonctionnement, de façon à faire tourner synchroniquement le clou de fixation (1) emboîté à l'extrémité avant du câble de convoyage (310), puis à entraîner le câble pour libérer le clou de fixation (1) dans une seconde condition de fonctionnement.
